# EUROPEAN PATENT APPLICATION

(11) **EP 3 406 228 A1**
(43) Date of publication of application: **28.11.2018**
(21) Application number: 18166165.3
(22) Date of filing: 06.04.2018
(51) Int. Cl.: A61F 2/966

(54) **STENT DELIVERY SYSTEM**

(30) Priority: 11.04.2017 US 201762484184 P
(71) Applicant: Cook Medical Technologies LLC, Bloomington, IN 47404 (US)
(72) Inventor: LONN, Melissa, Lafayette, IN Indiana 47906 (US)
(74) Representative: Garratt, Peter Douglas

(57) **Abstract**

A stent delivery system may include a stent disposed between an inner tubular member and an outer tubular member as part of a pre-delivery package for the stent. Once the pre-delivery package is disposed over a wire guide, the inner tubular member may be withdrawn through an opening in a pusher, which is then used to push the stent through a catheter lumen to a treatment site.

## Description

### TECHNICAL FIELD

The present invention relates generally to medical devices and more particularly to stent delivery systems.

### BACKGROUND

Stents are relatively small tubes that are typically used in various medical procedures to keep a certain bodily structure open. In some medical procedures, implantation of the stent is the end goal or primary objective of the medical procedure. In other medical procedures, the stent is implanted so that a subsequent procedure can be performed. Treatment of intracranial aneurysms is one area where stent implantation is performed. Where an aneurysm forms in a blood vessel or artery, a stent may be implanted adjacent to the aneurysm. For some treatments, the stent may be implanted in order to divert blood flowing through the vessel/artery away from the aneurysm. For other treatments, a stent may be implanted as part of an aneurysmal coiling operation, which involves inserting a coil into the aneurysm, which in turn may cause the aneurysm to clot. A catheter is used to deliver the coil to the aneurysm. In situations where the neck of the aneurysm is relatively wide, the stent may be implanted adjacent to the aneurysm in order to prevent the coil from falling out of the aneurysm. The wall of the stent may have one or more openings, and so after the stent is implanted, the coil and/or the catheter delivering the coil may be moved through an opening in order to insert the coil into the aneurysm.

The path to the intracranial aneurysm can be long and tortuous, with the entry point for the medical devices often being at the patient's groin. In addition to being long and tortuous, the vessels/arteries making up the path can be delicate and easy to puncture or damage. As such, a balance between flexibility and stiffness is needed in order to successfully traverse the path to the aneurysm while avoiding damage to the vessels or arteries. At the same time, the sizes of the vessels/arteries can be extremely small, especially as the path moves into the brain, where the sizes of the vessels or arteries can be around 2 millimeters or smaller. Consequently, delivery devices that deliver the stents need to have a very small profile, such as on the order of 3 French (1 millimeter) or smaller.

Current stent delivery devices used to treat intracranial aneurysms, or other neurovascular devices with comparably small profiles, such as stent retrievers or thrombectomy devices, utilize two wire guides. A first wire guide is used with a microcatheter to reach the access site. The first wire guide is then removed, and a stent attached to a second wire guide is inserted through the catheter to the treatment site. The stent in combination with the second wire guide may be considered a stent delivery package.

However, due to the long and tortuous path, simply gaining access to and/or reaching the treatment site with the first wire guide and the catheter can be difficult, and removing the first wire guide so that the stent delivery package can be inserted into the catheter can be cumbersome and time consuming. Also, removing the first wire guide from the long and tortuous path can cause the catheter to move, resulting in a loss of access to the treatment site, which was already difficult to obtain in the first place. Thus, improved stent delivery systems that reduce the time needed to deliver the stents, make the delivery less cumbersome, and reduce the likelihood that access is lost may be desirable.

### BRIEF DESCRIPTION OF THE DRAWINGS

Fig. 1 is a cross-sectional side view of a stent delivery system configured to deliver a stent to a treatment site within a patient.
Fig. 2 is a cross-sectional side view of a pre-delivery package and a pusher of the stent delivery system of Fig. 1, with the pusher engaged with the stent, and a proximal portion of an inner tubular member of the pre-delivery package inserted through a pusher opening.
Fig. 3 is a cross-sectional side view of the stent delivery system of Fig. 1, with the pre-delivery package and a distal portion of the pusher positioned over a proximal portion of a wire guide of the stent delivery system.
Fig. 4 is a cross-sectional side view of the stent delivery system of Fig. 1, with the inner tubular member being completely removed from the pre-delivery package.
Fig. 5 is a cross-sectional side view of the stent delivery system of Fig. 1, with the pusher having distally advanced the stent and an outer tubular member of the pre-delivery package over the wire guide into an opening of a proximal hub.
Fig. 6 is a cross-sectional side view of the stent delivery system of Fig. 1, with the pusher having pushed the stent through a catheter lumen to the treatment site.
Fig. 7 is a cross-sectional side view of an alternative pusher for the stent delivery system of Fig. 1.

### DETAILED DESCRIPTION

### Overview

By way of introduction, the below embodiments relate to medical systems, devices, and methods for delivering a stent to a treatment site within a patient. In one embodiment, a stent delivery system includes: a stent; a wire guide, and a tubular member. The wire guide longitudinally extends from a proximal portion to a distal portion, and is configured to deliver the stent from the proximal portion to the distal portion. The tubular member includes an outer surface in releasable engagement with an inner surface of the stent, and an inner surface defining a lumen sized to permit a proximal portion of the wire guide to be disposed therein. The tubular member is configured to be disengaged from the stent when the proximal portion of the wire guide is disposed within the lumen.

In some embodiments, a pusher is configured to distally push the stent over the wire guide. The pusher includes a pusher opening through which the tubular member is configured to move to be disengaged from the stent.

In some embodiments, the tubular member includes a proximal portion that extends through the pusher opening to outside of the pusher when the pusher is engaged with the stent.

In some embodiments, the pusher includes a tubular body and a pusher lumen extending through the tubular body. The tubular body is configured to be disposed over the wire guide and engage with the stent to distally push the stent.

In some embodiments, the pusher opening is a proximal opening of the pusher lumen.

In some embodiments, the pusher includes an elongate pusher rod connected to a proximal portion of the tubular body of the pusher.

In some embodiments, the pusher opening is a side opening of the tubular body.

In some embodiments, the tubular member is an inner tubular member, and the stent delivery system further includes: an outer tubular member that includes an inner surface in releasable engagement with an outer surface of the stent, and a catheter that includes a proximal hub configured to engage with the outer tubular member to disengage the inner surface of the outer tubular member from the outer surface of the stent.

In a second embodiment, a stent delivery system includes a stent, a pre-delivery package for the stent, a catheter, and a pusher. The pre-delivery package for the stent includes an inner tubular member over which the stent is disposed, and an outer tubular member disposed over the stent. The catheter includes a catheter body longitudinally extending from a proximal portion to a distal portion, and a catheter lumen longitudinally extending through the catheter body. The catheter lumen is configured to deliver the stent to a treatment site within a patient near the distal portion. In addition, the pusher is configured to engage with the stent, where the pusher includes a pusher opening though which a proximal portion of the inner tubular member of the pre-delivery package extends.

In some embodiments, the catheter includes a proximal hub configured to engage with the outer tubular member to prevent the outer tubular member from entering into the lumen of the catheter.

In some embodiments, a proximal portion of the outer tubular member is configured to proximally extend past a proximal end of the stent to create an engagement region within the outer tubular member. A distal end of the pusher is configured to engage with the stent when disposed in the engagement region.

In some embodiments, a wire guide is configured to be movably disposed within the catheter lumen. The stent is configured to be disposed over the wire guide for delivery to the treatment site, and the pusher is configured to distally push the stent over the wire guide to deliver the stent to the treatment site.

In some embodiments, the pusher includes a tubular body and a pusher lumen extending through the tubular body, where the tubular body is configured to be disposed over the wire guide and engage with the stent to distally push the stent to the treatment site.

In some embodiments, the pusher opening is a proximal opening of the pusher lumen.

In some embodiments, the pusher includes an elongate pusher rod connected to a proximal portion of the tubular body of the pusher.

In some embodiments, the pusher opening comprises a side opening of the tubular body.

In some embodiments, the wire guide includes a first portion and a second portion distal the first portion, where a diameter of the first portion is smaller than an inner diameter of the inner tubular member, and where a diameter of the second portion is larger than the inner diameter of the inner tubular member of the pre-delivery package.

In some embodiments, a distal end of the inner tubular member extends past a distal end of the stent.

In another embodiment, a stent delivery method is performed. The method includes: disposing a tubular member over a proximal portion of a wire guide, an outer surface of the tubular member attached to inner surface of a stent; with the tubular member disposed over the proximal portion of the wire guide, biasing the tubular member to detach the tubular member from the stent; engaging a pusher with the stent; and with the pusher engaged with the stent, distally advancing the stent over the wire guide to a treatment site within a patient.

In some embodiments, the pusher includes a pusher opening, and the method includes: moving the tubular member through the pusher opening in response to biasing the tubular member.

In some embodiments, the method further includes: with the pusher engaged with the stent, maintaining a longitudinal position of the stent relative to the wire guide while biasing the tubular member to detach the tubular member from the stent.

In some embodiments, the tubular member comprises an inner tubular member, and the method further includes: with the pusher engaged with the stent, engaging a proximal hub of a catheter with an outer tubular member. An inner surface of the outer tubular member is engaged with an outer surface of the stent, and engaging the pusher with the stent in combination with engaging the proximal hub of the catheter with the outer tubular member disengages the inner surface of the outer tubular member from the outer surface of the stent.

Other embodiments are possible, and each of the embodiments can be used alone or together in combination. Accordingly, various embodiments will now be described with reference to the attached drawings.

### Embodiments

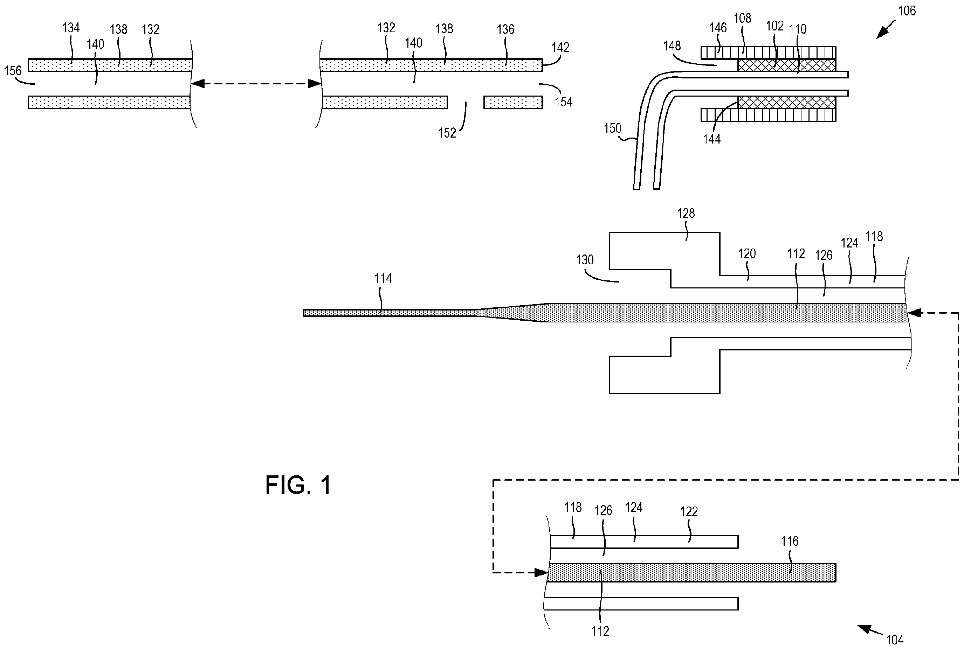

Fig. 1 shows a cross-sectional side view of a stent delivery system configured to deliver a stent 102 to a treatment site 104 within a patient. The stent 102 may be of any type, including expandable and non-expandable, that is suitable for a corresponding medical procedure. The treatment site 104 may be any location where the stent 102 may be delivered and/or implanted. In one non-limiting example application, the treatment site 104 may be a blood vessel in a patient's brain where an aneurysm has formed, and the stent 102 may be a self-expandable stent used in the treatment of the aneurysm.

Prior to being used in a medical procedure and/or delivered to the treatment site 104, the stent 102 may be included in and/or attached to a pre-delivery package 106. The pre-delivery package 106 for the stent 102 may include an outer tubular member (or outer transfer tube) 108 and an inner tubular member (or inner transfer tube) 110. As shown in Fig. 1, an inner surface of the outer tubular member 108 may be attached to an outer surface of the stent 102, and an outer surface of the inner tubular member 110 may be attached to an inner surface of the stent 102. Otherwise stated, the stent 102 may be disposed about or over the inner tubular member 110, and the outer tubular member 108 may be disposed about or over the stent 102. Each of the outer tubular member 108 and the inner tubular member 110 may be in releasable engagement with the stent 102, meaning that although the outer tubular member 108 and the inner tubular member 110 are engaged with, in contact with, and/or attached to the stent 102 as part of the pre-delivery package 106, each of the outer tubular member 108 and the inner tubular member 110 may be configured to be detached and/or disengaged from stent 102, as described in further detail below.

In some example configurations, the stent 102 may be crimped to the inner tubular member 110 in order to be attached to the inner tubular member 110. The stent 102 and the inner tubular member 110 may then be inserted into the outer tubular member 108, such as through use of a pusher 132, which is described in further detail below.

The stent delivery system may further include an elongate wire guide 112 longitudinally extending from a proximal portion 114 to a distal portion 116. The stent 102 may be movably disposed about the wire guide 112 in order to be delivered to the treatment site 104, as described in further detail below. The stent delivery system may also include an elongate catheter 118 (which in some example embodiments may be referred to as a microcatheter) that longitudinally extends from a proximal portion 120 to a distal portion 122. (The drawings are not drawn to scale, and for illustration purposes only, the distal portion of the stent delivery system near the treatment site 104 is illustrated as dimensionally smaller than the proximal portion of the stent delivery system). The catheter 118 may include a catheter body 124 extending from the proximal portion 120 to the distal portion 122, and a catheter lumen 126 longitudinally extending through the catheter body 124. A proximal hub 128 may be coupled to or be considered part of the proximal portion 120 of the catheter 118. An opening 130 of the proximal hub 128 may be in fluid communication with the catheter lumen 126.

Fig. 1 shows the wire guide 112 as being movably disposed within the catheter lumen 126. Initially, the wire guide 112 may be inserted into the patient and distally moved through a path within the patient until the distal portion 116 of the wire guide 112 reaches the treatment site 104. The catheter lumen 126 may then be inserted over the wire guide 112, and the catheter 118 may be distally advanced until the distal portion 122 of the catheter 118 reaches the treatment site 104. Thereafter, medical devices to be delivered to the treatment site 104, such as the stent 102, may be inserted and distally advanced over the wire guide 112, through the hub opening 130 and the catheter lumen 126, until the medical device reaches the treatment site 104.

In addition, the stent delivery system may include an elongate pusher 132 that longitudinally extends from a proximal portion 134 to a distal portion 136. In one example embodiment, as shown in Fig. 1, the pusher 132 may include a tubular body 138 extending from the proximal portion 134 to the distal portion 136, and a pusher lumen 140 longitudinally extending through the tubular body 138.

A distal end 142 of the pusher 132 may be configured to engage with a proximal end 144 of the stent 102 to distally advance the stent 102 over the wire guide 112 to the treatment site 104. Accordingly, the pusher body 138 may be made of a material and have a sufficient thickness that provides sufficient strength to push the stent 102, while still being flexible enough to move through a tortuous path in the body. In some example configurations, the thickness of the pusher body 138 may be about equal to the thickness of the stent 102. Also, example materials for the pusher body 138 may include Nitinol or another polymer, although other materials may be possible.

In addition, in one example configuration, as shown in Fig. 1, a proximal portion 146 of the outer tubular member 108 may proximally extend past the proximal end 144 of the stent 102, creating an engagement region 148 within the outer tubular member 108. The engagement region 148 may be used to facilitate engagement between the distal end 142 of the pusher 132 and the proximal end 144 of the stent 102. Accordingly, the stent 102, the outer tubular member 108, the inner tubular member 110, and the pusher 132 may be sized relative to each other so that the distal portion 136 of the pusher 132 can be inserted into the engagement region 148 to engage with the stent 102.

In addition, as shown in Fig. 1, the inner tubular member 110 may include a proximal portion 150 that proximally extends past the proximal portion 146 of the outer tubular member 108. The proximal portion 150 may be configured to be biased in order to detach the inner tubular member 110 from the stent 110 and proximally moved relative to the stent 102 so that stent 102 is no longer disposed over the inner tubular member 110.

While the bias is applied to the proximal portion 150 of the inner tubular member 110, the distal end 142 of the pusher 132 may be configured to be engaged with the proximal end 144 of the stent 102 and maintain the stent 102 in its present position in order to prevent the stent 102 from proximally moving with the inner tubular member 110. This may allow the inner tubular member 110 to detach from the stent 102.

Additionally, the pusher 132 may include a pusher opening 152 that is configured to allow the inner tubular member 110 to be inserted into and moved therethrough. As described in further detail below, the inner tubular member 110, starting with its proximal portion 150, may be inserted into a distal opening 154 of the pusher lumen 140 and then moved through the pusher opening 152. This may initially allow the distal portion 150 of the pusher to not impede the ability of the distal end 142 of the pusher 132 to engage with the proximal end 144 of the stent 102 in the engagement region 148. The proximal portion 150 may be long enough so that when the distal end 142 of the pusher 132 is engaged with the proximal end 144 of the stent 102, at least part of the proximal portion 150 may extend through the pusher opening 152 and disposed outside of the pusher 132. An operator of the stent delivery system, such as a physician, or a machine configured to grasp the distal portion 150, may be configured to pull on or otherwise bias the proximal portion 150 to detach the inner tubular member 110 from the stent 102. Upon detachment, the proximal portion 150 may continue to be biased until a remaining part of the inner tubular member 110 is no longer within the stent 102 and/or moved through the pusher opening 152 to outside of the pusher 132.

In the configuration shown in Fig. 1, the pusher opening 152 is shown as being a side opening extending through the tubular body 138 at the distal portion 136. In other example configurations, the pusher opening 152 may be a side opening disposed at the proximal portion 134 of the pusher 132. In still other example configurations, the pusher 132 may not include a side opening, and the pusher opening may be a proximal opening 156 of the pusher lumen 140. The longitudinal length of the proximal portion 150 of the inner tubular member 110 may be sized appropriately to accommodate where the pusher opening 152 is located in the pusher 132.

Further details of the stent delivery system are now described with reference to a method of operating the stent delivery system to deliver the stent 102 to the treatment site 104.

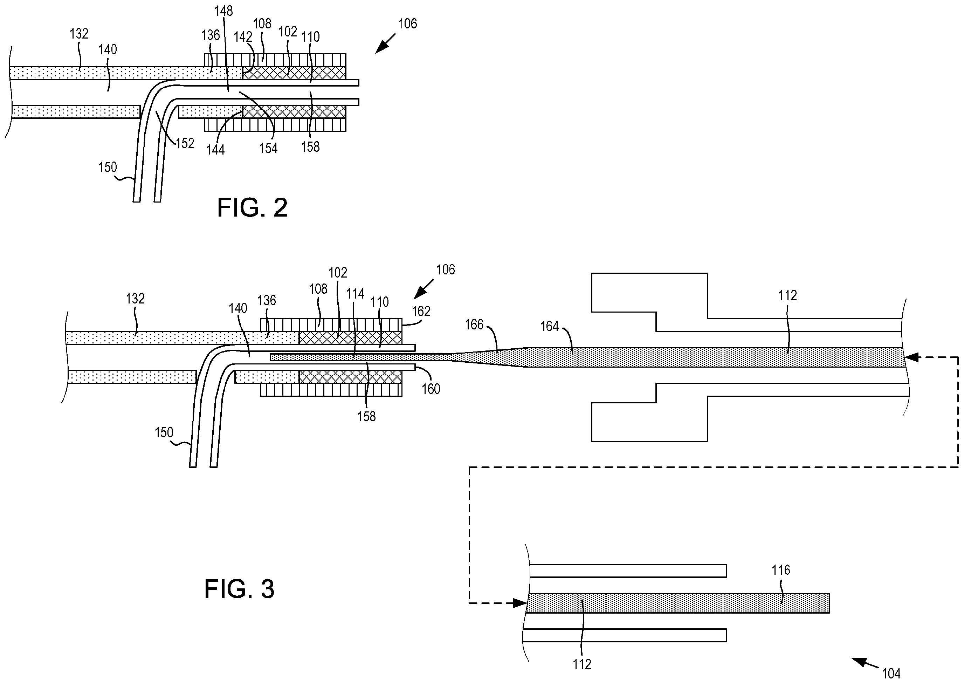

Referring to Fig. 2, the pre-delivery package 106 and the pusher 132 may be moved relative to each other so that the proximal portion 150 of the inner tubular member 110 is inserted into the distal opening 154 of the pusher lumen 140 and then moved through the pusher opening 152. The distal portion 136 of the pusher 132 may be inserted into the engagement region 148 until the distal end 142 of the pusher 132 engages with the proximal end 144 of the stent 102. In doing so, more and more of the proximal portion 152 may be moved through the distal opening 154 and the pusher opening 152. When the distal end 142 of the pusher 132 engages with the proximal end 144 of the stent 102, a certain length of the proximal portion 150 may have moved through the pusher opening 152 and be disposed outside of the pusher 132. Also, when the proximal end 144 of the stent 102 and the distal end 142 of the pusher 132 are engaged, a central lumen 158 of the stent 102 (or of the inner tubular member 110 or generally of the pre-delivery package 106) may be coaxially aligned with the pusher lumen 140, which may enable the pusher 132 to distally advance the stent 102 over the wire guide 112, as described in further detail below.

Referring to Fig. 3, the pre-delivery package 106 and the distal portion 136 of the pusher 132 may be positioned over the proximal portion 114 of the wire guide 112 so that the proximal portion 114 is disposed in the central lumen 158, and in some example methods, extends into the pusher lumen 140, as shown in Fig. 3. In some example configurations, a distal end 160 of the inner tubular member 110 may distally extend past a distal end 162 of the stent 102, which may facilitate the insertion of the proximal portion 114 of the wire guide 112 into the central lumen 158.

Additionally, as shown in Figs. 1-3, for some example configurations, the proximal portion 114 of the wire guide 112 may have a diameter that is smaller than a diameter of a portion 164 of the wire guide 112 distal the proximal portion 114. The portion 164 with the larger diameter may distally extend all the way to the distal portion 116 of the wire guide 112, although other configurations that utilize a wire guide with varying diameters over a portion of the wire guide 112 that is distal the proximal portion 114 may be possible. Also, as shown in Figs 1-3, the wire guide 112 may include a taper region 166 that provides a transition between the smaller-diameter proximal portion 114 and the larger-diameter distal portion 164.

The wire guide 112 may have a reduced diameter at the proximal portion 114 in order to avoid having to use a smaller-sized wire guide or a larger profile catheter. That is, the catheter 118 and the wire guide 112 may be optimally sized for a given medical procedure such that the wire guide 112 and the catheter 118 have an optimal balance between flexibility and stiffness in order to traverse a possibly long and tortuous path within the patient to the treatment site 104, and also such that the stent 102, the catheter 118, and the wire guide 112 may be moved within relatively small passageways, such as intracranial blood vessels. However, by including the inner tubular member 110 within the stent 102, the central lumen 158 may have a reduced diameter compared to other embodiments where the inner tubular member 110 is not included with the pre-delivery package 106. That smaller diameter may be smaller than the diameter of an untapered wire guide, which would prevent the untapered wire guide from being able to be inserted into the inner tubular member 110.

It may be undesirable to simply use a smaller-sized wire guide because such a wire guide may lack the necessary stiffness to traverse the path within the patient to the treatment site 104. Also, using a larger sized catheter 118 may allow for a larger inner tubular member to be used, but doing so may be unsuitable for the small size of the passageways through which the catheter 118 will be moved. Accordingly, by tapering the wire guide 112 at the proximal portion 114, the proximal portion 114 may be sized to have a diameter that is smaller than the diameter of the central lumen 158 in order to be able to be inserted into the central lumen 158. As a result, a smaller-sized wire guide or a larger catheter 118 do not have to be used. In one example application where the stent delivery system is for treating intracranial aneurysms, the wire guide 112 may have a .014 inch diameter except at the distal portion 114, which may be tapered down to .010 inches to accommodate the smaller-sized central lumen 158.

Also, including the inner tubular member 110 with the pre-delivery package 106 may provide a layer of protection between the proximal portion 114 of the wire guide 112 and the stent 102. Additionally, the inner tubular member 110 may facilitate or guide the proximal portion 114 of the wire guide 112 into the central lumen 156. That is, if the inner tubular member 110 was not included with the pre-delivery package 106, insertion of the proximal portion 114 into the central lumen 158 may be more difficult due to a tendency of the proximal portion 114 to snag or get caught on the stent wall, which in turn may lead to the proximal portion 114 damaging the stent 102. Other pre-delivery packages may not include an inner tubular member, and instead use a second wire guide as part of the pre-delivery package. However, as previously mentioned, such stent delivery systems that utilize a second wire guide may provide a means for stent delivery that is time consuming, cumbersome, and that has a high risk of loss of access to the treatment site. Inclusion of the inner tubular member 110 serves to eliminate the need for a second wire guide, and in doing so, facilitates the loading of the stent 102 onto the single wire guide 112 while protecting against the possible damage that the wire guide 112 may cause to the stent 102.

Nevertheless, it may be desirable for the reduction in size of the central lumen 158 due to the inclusion of the inner tubular member 110 to be as minimal as possible. In that regard, the inner tubular member 110 may be made of a thin material, such as polyimide or other similar material.

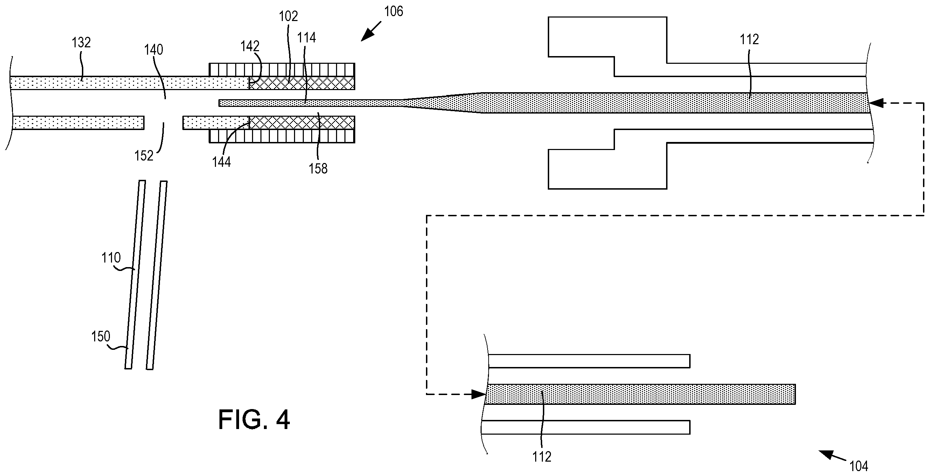

Referring to Fig. 4, with the proximal portion 114 disposed in the central lumen 158 and the distal end 142 of the pusher 132 engaged with the proximal end 144 of the stent 102, a bias may be applied to the proximal portion 150 of the tubular member 110 to detach the outer surface of the inner tubular member 110 from the inner surface of the stent 102 and move the inner tubular member 110 through the pusher opening 152 until the inner tubular member 110 is completely removed from the pre-delivery package 106. An operator, such as a physician, or a machine configured to grasp the proximal portion 150 may apply the bias. By removing the inner tubular member 110, the pusher lumen 140 may be uninhibited from distally advancing over the wire guide 112. Also, by engaging the distal end 142 of the pusher 132 with the proximal end 144 of the stent 102, the pusher 132 may maintain the stent 102 in its longitudinal position relative to the wire guide 112 and allow the inner tubular member 110 to be detached from the stent 102.

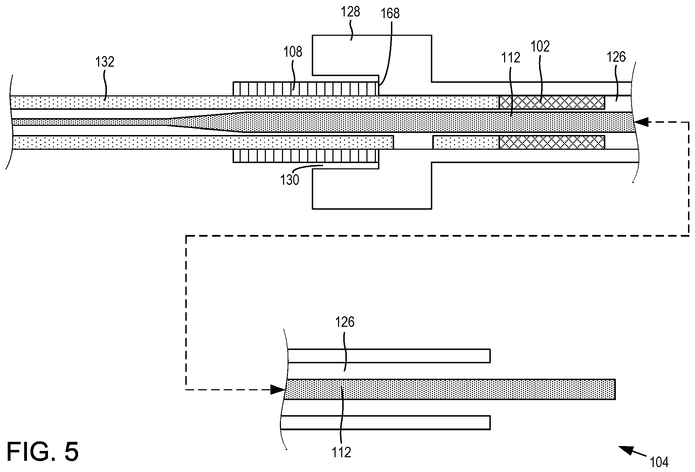

Referring to Fig. 5, with the inner tubular member 110 removed, the pusher 132 may continue to distally advance the stent 102 and the outer tubular member 108 over the wire guide 112 into the opening 130 of the proximal hub 128. The opening 130 of the proximal hub 128 may be large enough to receive both the stent 102 and the outer tubular member 108. However, the catheter lumen 126 may be sized to be able to receive the stent 102 but not both the stent 102 and the outer tubular member 108. As such, at the point where the hub opening 130 and the catheter lumen 126 meet, the outer tubular member 108 may encounter a side wall 168 of the proximal hub 128, which may prevent the outer tubular member 108 from distally advancing any further. The pusher 132 may continue to distally bias the stent 102, which in turn may cause the outer surface of the stent 102 to detach or separate from the inner surface of the outer tubular member 108, and the pusher 132 and the stent 102 may continue to distally advance over the wire guide 112 through the catheter lumen 126, which is what is shown in Fig. 5. In some example methods, the outer tubular member 108 may be removed from the proximal hub 128 once it is detached from or no longer in contact with the stent 102.

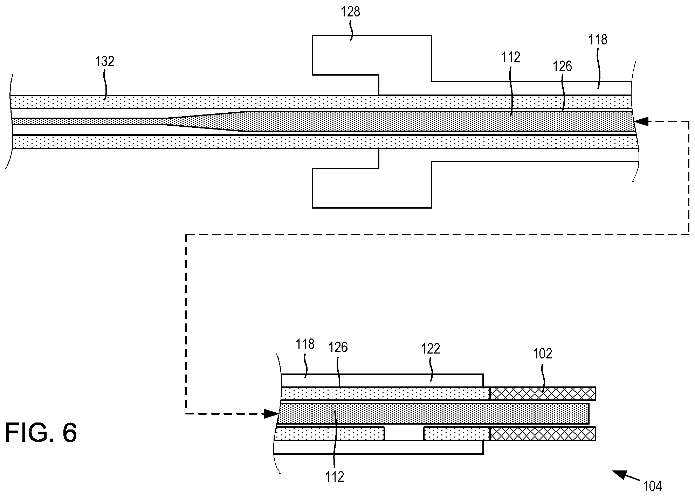

Referring to Fig. 6, the pusher 132 may continue to distally bias the stent 102 over the wire guide 112 to push the stent 102 through the catheter lumen 126 to the distal portion 122 of the catheter 118, until the stent 102 reaches the treatment site 104. Although not shown, once the stent 102 is positioned as desired at the treatment site 104, one or more of the wire guide 112, the catheter 118, and/or the pusher 132 may be proximally retracted and withdrawn from the patient.

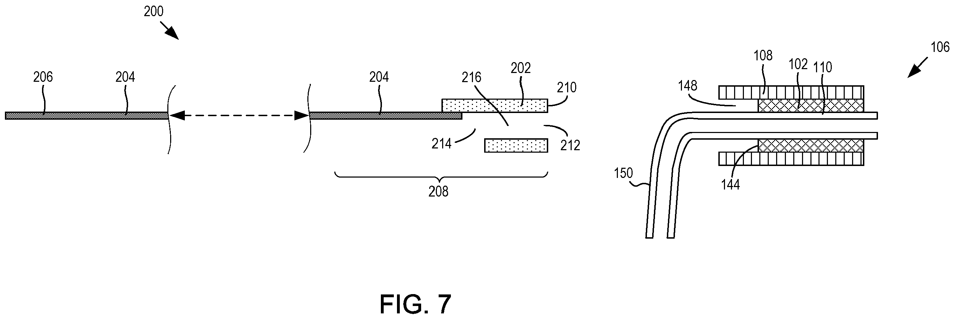

Fig. 7 shows a cross-sectional side view of an alternative pusher 200 that may be used instead of the pusher 132 with the other components of the stent delivery system. As previously described with reference to Figs. 1-6, the first pusher 132 includes an elongate tubular body 138 that extends from the proximal portion 134 to the distal portion 136. In contrast, the second pusher 200 in Fig. 7 may include a distal cannula or ring 202 connected to an elongate pusher rod 204 that extends from a proximal portion 206 to a distal portion 208 of the pusher 200. Similar to the first pusher 132, a distal end 210 of the distal cannula 202 may be configured to be inserted to the engagement region 148 and engage with the proximal end 144 of the stent 102. The inner tubular member 110 may be inserted through a distal opening 212 of the cannula 202 and then moved through a proximal opening 214 of the distal cannula 202. In this regard, the proximal opening 214 serves as the pusher opening for the second pusher 200.

Also, after the inner tubular member 110 is detached from the stent 102 and the pusher 200 continues to distally advance the stent 102 over the wire guide 112, a lumen 216 of the cannula 202 may be disposed over or about the wire guide 112, but the portion of the wire guide 112 proximal the lumen 216 may have exited the proximal opening 214 and extend along side the pusher rod 204. In contrast, with the first pusher 132, the wire guide 112 may have proximally extended through the pusher lumen 140 until it reached the proximal opening 156. The second pusher 200 shown in Fig. 7 may be similar and/or correspond to a rapid exchange type configuration, where a length of the wire guide 112 needed for delivery of the stent 102 when the second pusher 200 is used may be less than, such as half, the length that is needed for the wire guide 112 when the first pusher 132 is used.

In sum, the stent delivery system embodiments and related methods shown and described with reference to Figs. 1-7 may use only a single wire guide to deliver a stent to a treatment site. Such a delivery system may be in contrast to one that uses two wire guides, such as one where the second wire guide is part of the pre-delivery package for the stent. Through use of only a single wire guide, the cumbersome, timely, and loss of access problems that accompany having to use two wire guides may be eliminated. Although not limited, such a configuration may be particularly useful in neurovascular medical procedures, especially for those involving the treatment of intracranial aneurysms.

The foregoing description of various embodiments of the invention has been presented for purposes of illustration and description. It is not intended to be exhaustive or to limit the invention to the precise embodiments disclosed. Numerous modifications or variations are possible in light of the above teachings. The embodiments discussed were chosen and described to provide the best illustration of the principles of the invention and its practical application to thereby enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally, and equitably entitled.

## Claims

1. A stent delivery system comprising:
a stent;
a wire guide longitudinally extending from a proximal portion to a distal portion, the wire guide configured to deliver the stent from the proximal portion to the distal portion; and
a tubular member comprising:
an outer surface in releasable engagement with an inner surface of the stent; and
an inner surface defining a lumen sized to permit a proximal portion of the wire guide to be disposed therein,
wherein the tubular member is configured to be disengaged from the stent when the proximal portion of the wire guide is disposed within the lumen.

2. The stent delivery system of claim 1, further comprising:
a pusher configured to distally push the stent over the wire guide, wherein the pusher comprises a pusher opening through which the tubular member is configured to move to be disengaged from the stent.

3. The stent delivery system of claim 2, wherein the tubular member comprises a proximal portion that extends through the pusher opening to outside of the pusher when the pusher is engaged with the stent.

4. The stent delivery system of claim 2 or claim 3, wherein the pusher comprises a tubular body and a pusher lumen extending through the tubular body, wherein the tubular body is configured to be disposed over the wire guide and engage with the stent to distally push the stent.

5. The stent delivery system of any one of claim 2 to claim 4, wherein the pusher opening is a proximal opening of the pusher lumen.

6. The stent delivery system of claim 5, wherein the pusher further comprises an elongate pusher rod connected to a proximal portion of the tubular body of the pusher.

7. The stent delivery system of any one of claim 2 to claim 4, wherein the pusher opening is a side opening of the tubular body.

8. The stent delivery system of any of claims 1 to 7, wherein the tubular member comprises an inner tubular member, and wherein the stent delivery system further comprises:
an outer tubular member comprising an inner surface in releasable engagement with an outer surface of the stent; and
a catheter comprising a proximal hub configured to engage with the outer tubular member to disengage the inner surface of the outer tubular member from the outer surface of the stent.

9. A stent delivery system kit comprising:
a stent;
a pre-delivery package for the stent, the pre-delivery package comprising:
an inner tubular member over which the stent is disposed; and
an outer tubular member disposed over the stent;
a catheter comprising:
a catheter body longitudinally extending from a proximal portion to a distal portion; and
a catheter lumen longitudinally extending through the catheter body, the catheter lumen configured to deliver the stent to a treatment site within a patient near the distal portion; and
a pusher configured to engage with the stent, wherein the pusher comprises a pusher opening though which a proximal portion of the inner tubular member of the pre-delivery package extends.

10. The stent delivery system of claim 9, wherein the catheter further comprises a proximal hub configured to engage with the outer tubular member to prevent the outer tubular member from entering into the lumen of the catheter.

11. The stent delivery system of claims 9 or 10, wherein a proximal portion of the outer tubular member is configured to proximally extend past a proximal end of the stent to create an engagement region within the outer tubular member, and wherein a distal end of the pusher is configured to engage with the stent when disposed in the engagement region.

12. The stent delivery system of any of claims 9 to 11, further comprising:
a wire guide configured to be movably disposed within the catheter lumen,
wherein the stent is configured to be disposed over the wire guide for delivery to the treatment site, and
wherein the pusher is configured to distally push the stent over the wire guide to deliver the stent to the treatment site.

13. The stent delivery system of claim 12, wherein the pusher comprises a tubular body and a pusher lumen extending through the tubular body, wherein the tubular body is configured to be disposed over the wire guide and engage with the stent to distally push the stent to the treatment site, and
wherein the pusher opening is a proximal opening of the pusher lumen or comprises a side opening of the tubular body.

14. The stent delivery system of claims 12 or 13, wherein the wire guide comprises a first portion and a second portion distal the first portion, wherein a diameter of the first portion is smaller than an inner diameter of the inner tubular member, and wherein a diameter of the second portion is larger than the inner diameter of the inner tubular member of the pre-delivery package.

15. The stent delivery system of any of claims 12 to 14, wherein a distal end of the inner tubular member extends past a distal end of the stent.
